# EUROPÄISCHE PATENTSCHRIFT

(11) **EP 0 582 055 B1**
(45) Veröffentlichungstag und Bekanntmachung des Hinweises auf die Patenterteilung: **08.03.2006**
(21) Anmeldenummer: 93108504.7
(22) Anmeldetag: 26.05.1993
(51) Int. Cl.: A61K 31/025, A61P 27/02

(54) **Perfluorkohlenwasserstoffe als Tamponade für den Glaskörperraum des Auges**
Perfluorocarbons as tamponade for the vitreous body cavity of the eye
Perfluorocarbones comme tampon pour la cavité du corps vitreux oculaire

(30) Priorität: 25.06.1992 DE 4220882
(43) Veröffentlichungstag der Anmeldung: 09.02.1994
(73) Patentinhaber: Chiron Adatomed Pharmazeutische und Medizintechnische Gesellschaft mbH, 85609 Dornach (DE)
(72) Erfinder: Meinert, Hasso, Prof.Dr., W-7910 Neu-Ulm (DE); Heimann, Klaus, Prof.Dr.med., W-5000 Köln 41 (DE); Kreiner, Christine F., Dr., W-8000 München 82 (DE); Kirchhof, Bernd, Prof.Dr.med., W-5000 Köln 40 (DE)
(74) Vertreter: Maiwald, Walter

(56) Entgegenhaltungen:
- EP-A- 0 493 677
- WO-A-83/03201
- WO-A-92/16240
- INVEST. OPHTHALMOL. Bd. 13, Nr. 8, 1974, Seiten 627 - 629 I.J. CONSTABLE 'Perfluoropentane in experimental ocular surgery'
- OPHTHALMOLOGY Bd. 92, Nr. 5, 1985, Seiten 651 - 656 S. CHANG ET AL. 'Perfluorocarbon gases in vitreous surgery'
- BULL. SOC. BELGE OPHTHALMOL. Bd. 238, 1990, Seiten 145 - 150 C. CLAES ET AL. 'The use of perfluorocarbon liquids in vitreous surgery'

## Beschreibung

Die Erfindung betrifft die Verwendung von Perfluorkarbon als Tamponade des Glaskörperraums eines Auges, insbesondere zur Behandlung von Netzhautablösung.

Aus Investigative Ophthalmology, Vol. 13/8, 1974, Seiten 627-629 ist es bekannt, Perfluoropentan in den Glaskörperraum eines nichtvitrektomierten Auges zu injizieren, wobei durch allmähliche Gasbildung bei Raumtemperatur maximal etwa 20% des Glaskörperraumes von dem Gasvolumen ausgefüllt werden. Eine Tamponade des gesamten Glaskörperraumes wird hierdurch nicht erreicht.

Weiterer relevante Stand der Technik ist in der folgenden Literatur beschrieben:
Aronowitz JD and Brubaker RF; Effect of intraocular gas on intraocular pressure; Arch Ophthalmol, 94 (1976) 1191-6
Cibis PA, Becker B, Okun E and Canaan S; The use of liquid silicone.in retinal detachment; Arch Ophthalmol, 68 (1962) 590-9
Custodis E; Die Behandlung des Netzhautablösung durch umschriebene Diathermiekoagulation und einer mittels Plombenaufnähung erzeugten Eindellung der Sklera im Bereich des Risses; Klin Monatsbl Augenheilkd, 129 (1956) 476-95
Dimopoulos S and Heimann K; Spätkomplikationen nach Silikon-ölinjektion. Langzeitbeobachtung an 100 Fällen; Klin Monatsbl Augenheilkd, 189 (1986) 223-7
Freeman WU, Lipson BK, Morgan CM and Liggett PE; New posteriorly located retinal breaks after pneumatic retinopexy; Ophthalmology, 95 (1988) 14-8
Kanski JJ, Elkington AR and Daavies MS; Diplopia after retinal detachment surgery; Am J Ophthalmol, 76 (1973) 38-40
Lemmen KD, Dimopoulos S, Kirchhof B and Heimann K; Keratopathy following pars plana vitrectomy with silicone oil filling; Dev Ophthalmol, 13 (1987) 88-98
Lucke K and Laqua H; Silicone oil in the treatment of complicated retinal detachment; Springer, Berlin, Heidelberg, N.Y., 1990, 61-6
Lund OE and Pesch KJ; Über Früh- und Spätfolgen nach bulbusumschnürenden Operationen; Ber Dtsch Ophthal Ges, 67 (1965) 202-12
Machemer R, Buettner H, Norton EWD and Parel JM; Vitrectomy. A pars plana approach; Trans Am Acad Ophthalmol Otolanzngol, 75 (1971) 813-20
Norton EWD; Intraocular gas in the management of selected retinal detachments; Trans Am Ophthalmol Otolaryngol, 77 (1973) 85-98
Rubin ML; The induction refractive errors by retinal detachment surgery; Trans Am Ophthalmol Soc, 73 (1975) 452-90
Russo CE and Ruiz RS; Silicone sponge rejection. Early and late complications in retinal detachment surgery; Arch Ophthalmol, 85 (1971) 647-50
Stinson TW and Donlon JY; Interaction of intraocular air and sulfur hexafluoride with nitron oxide: a computer simulation; Anesthesiology, 56 (1982) 385-8.

Es sind somit zur Behandlung der Netzhautablösung im wesentlichen folgende Verfahren bekannt:
- Plomben oder Bänder werden auf das Auge aufgenäht. Damit wird die Traktion, die der Glasköper im Bereich des Netzhautloches verursacht, entspannt und das Foramen selber von außen tamponiert.
- Es werden Gase, wie SF₆, CF₄, C₂F₆, C₃F₈, Edelgase wie Krypton oder Xenon - Gastamponade - oder es werden Flüssigkeiten, z.B. Silikonöl - Flüssigkeitstamponade -, in den Glaskörperraum injiziert, wodurch eine innere Tamponade des Netzhautloches bewirkt wird.

Bei diesen beschriebenen Verfahren zur Netzhauttamponade treten eine Reihe auch aus obiger Literatur sich ergebende Schwierigkeiten auf:
a) Die eindellenden Operationen sind umfangreich. Es entstehen große Wundflächen, da die Bindehaut eröffnet und ein Plomben- oder Cerlage-Bett präpariert werden muß. Der Eingriff bedarf der retrobulbären Schmerzausschaltung oder der Intubationsnarkose und benötigt ca. 50 - 60 Minuten Zeit und ca. eine Woche stationären Krankenhausaufenthalt.
   Dabei besteht das Risiko der Einschränkung der postoperativen Augenbeweglichkeit mit konsekutiver Doppelbildwahrnehmung (Kanski et al., 1973).
   Weiterhin wird eine Fehlsichtigkeit (Kurzsichtigkeit, Stabsichtigkeit) induziert sowie verstärkt (Rubin, 1975).
   Die Durchblutung der Augenhülle (Aderhaut) wird behindert, darüberhinaus ist die Aderhaut Ausgangsort von Blutungen in das Augeninnere (Lund, 1965).
   Das Plomben- oder Bandmaterial kann abgestoßen werden, es kann sich infizieren und dann durch die Bindehaut nach außen spießen (Russo et al., 1971).
b) Die primäre Gastamponade ist zwangsläufig inkomplett, da mit Rücksicht auf den Augendruck nur maximal 1/8 Volumen des Glaskörperraumes tamponiert werden.
   Der Patient muß eine oft unbequeme Kopfhaltung ständig einhalten, damit die Gasblase vor das Netzhautloch gebracht wird.
   Die Gasblase expandiert aus bislang unbekannten Gründen auf das maximal Doppelte des injizierten Volumens (Aronowitz, 1976, Stinsen et al., 1982).
   Die hohe Beweglichkeit der inkompletten Tamponade gegen den gelförmigen Glaskörperrest induziert vitreo-retinale Spannungen, die in 10 - 24 % der Fälle sekundärer Netzhautlöcher meist in der unteren Zirkumfärenz induzieren (Freeman et al., 1988).
c) Die Tamponade durch Silikonöl setzt voraus, daß zuvor der Glaskörper im Rahmen eines operativen Eingriffes entfernt wird (Vitrektomie).
   Das Silikonöl behindert die Ernährung der Linse, so daß eine Katarakt entsteht (Dimopoulos et al., 1986).
   Das Silikonöl emulgiert und verursacht sekundäre Glaukome (Lucke et al., 1990). Es bedarf eines zweiten Eingriffes, um Silikonöl wieder aus dem Auge zu entfernen. Das Silikonöl induziert bei Kontakt irreversible Trübungen der Hornhaut (bandförmige Degeneration, Lemmen et al., 1987).

Aufgabe der Erfindung ist es, einen passageren und möglichst kompletten Glaskörperersatz zu schaffen, der ohne vorherige Glaskörperabsaugung injizierbar ist.

Diese Aufgabe wird erfindungsgemäß durch die kennzeichnenden Merkmale des Anspruchs 1 gelöst.

Hierdurch wird eine Glaskörpertamponade für ein Behandlungsverfahren, insbesondere der lochbedingten Netzhautablösung, geschaffen. Dadurch können ersetzt werden:
(a) die eindellende Operation (Plombenoperation nach Custodis, 1956)
(b) die direkte Gastamponade (Norton, 1973)
(c) die Silikonöltamponade (Cibis, 1962).

Es wird keine vorangehende Absaugung des Glaskörpers benötigt (Vitrektomie nach Machemer, 1971).

Man kann eine vollständige Tamponade des Glaskörperraums ohne vorherige Glaskörperabsaugung (Vitrektomie) und ohne die Injektion von Gasen erreichen. Das Perfluorcarbon (PFC) wird bei Raumtemperatur als Flüssigkeit in das Auge eingespritzt. Das Injektionsvolumen kann beim Menschen nur ca. 8 µl betragen, es füllt somit bei einem Gesamtvolumen des Glaskörpers von 4000 µl nur 0,2 % dieses Raumes aus. Im Glaskörperraum wird die PFC-Flüssigkeit (PFCL) auf 37°C Körpertemperatur erwärmt und geht allmählich in die Gasphase über. Aufgrund seines Dampfdruckes nimmt das PFCL beim Übergang in die Gasphase somit allmählich ein ca. 500-fach größeres Volumen als das Flüssigkeitsvolumen der primär injizierten Perfluorcarbon-Flüssigkeit ein. Das so injizierte Perfluorcaron kann als Gasblase über Wochen im Glaskörperraum verbleiben und wird allmählich über die vaskularisierten Augenhüllen resorbiert,

Der Glaskörperraum wird unter Verdrängung des Wasseranteils vollständig ausgefüllt. Der Injektionsort ist die Pars plana des Ziliarkörpers. Das allmähliche Verdampfen des PFCL erlaubt das Abpressen des Wassers aus dem Glaskörperraum (99% Wasseranteil), ohne das der Augendruck unverträglich hoch ansteigt.

Die Tamponade füllt schließlich den gesamten Glaskörperraum aus. Deshalb werden anders als mit der primären Glaskörperinjektionsmethode auch solche Netzhautlöcher tamponiert, die in der unteren Peripherie lokalisiert sind.

Dadurch wird die Indikation der Glaskörpertamponade entsprechend erweitert.

Nach fünf Wochen hat sich das zur Glaskörpertamponade eingesetzte Perfluorcarbon spontan resorbiert.

Es ist bekannt, daß reine Perfluorcarbone chemisch und physiologisch außerordentlich inerte Verbindungen sind (H. Meinert, EP Appl. No. 91120184.6). Perfluorcarbone besitzen sehr niedrige Grenzflächenspannungen (<30 dyn/cm) und relativ hohe Dichten (1,5 - 1,9 g/cm³) und hohe Dampfdrücke. PFCLs sind nicht löslich in Wasser. Mit zunehmendem Molekulargewicht steigen Siedepunkt und Schmelzpunkt. Umgekehrt nimmt der Dampfdruck ab.

PFCLs besitzen hohe Löslichkeiten für Gase, u.a. für O₂, CO₂ und SF₆. Die für die Augenheilkunde interessanten PFCLs sind farblose Flüssigkeiten, die sich auch bei einer Laserbehandlung vorzüglich eignen, weil sie dabei keiner Zersetzung unterliegen. Die Gaslöslichkeit entspricht dem Henryschen Gesetz, d.h. bei konstanter Temperatur ist die Löslichkeit proportional dem Partialdruck des Gases. Weiterhin hängt die Löslichkeit vom Molekulargewicht des zu lösenden Gases ab. Je höher das Molekulargewicht ist, desto höher ist die Löslichkeit im PFCL. Unter Normalbedingungen beträgt die Löslichkeit von O₂ 40 - 50 Vol%, die von CO₂ 100 - 150 Vol% und die von SF₆ 600 - 900 Vol% (bei geringer Abhängigkeit von dem jeweiligen PFCL). Aber auch gasförmige Perfluorcarbone, wie CF₄, C₂F₆, C₃F₈ und cycl. C F sind sehr gut löslich in flüssigen Perfluorcarbonen (PFCLs). Entsprechend den gegebenen physikalischen Bedingungen (Druck, Temperatur), der Konstitution des Gases und dem Anteil des zu lösenden Gases ergibt sich eine weitere Variation im Dampfdruck des Gemisches.

Deshalb kann der Gesamtdampfdruck des Systems in Abhängigkeit vom Eigendampfdruck des einzusetzenden PFCL, von dessen hoher Löslichkeit für Gase entsprechend dem Henryschen Gesetz und vom Molekulargericht des zu lösenden Gases, in einem wünschenswerten Bereich variiert werden.

Auch kann der sich nach der Injektion des PFCL ins Auge ergebende Dampfdruck allein schon von der Art her des verwendeten PFCL erreicht werden. Dabei werden bevorzugt als PFCLs Verbindungen mit Siedepunkten zwischen 40°C und 70°C vorgeschlagen. Zu diesen Verbindungen zählen vorzüglich Perfluorcarbone wie Perfluor-n-pentan, Perfluor-n-hexan, Perfluor-n-heptan und Perfluor-methylcyclopentan.

Der Vorteil bei diesen genannten PFCLs ist, daß sie unter den im Operationssaal gegebenen Bedingungen noch als Flüssigkeiten handhabbar sind und demzufolge auch genau dosiert injiziert werden können.

Dabei können die zu verwendenden PFCLs, wie schon ausgeführt, noch hinsichtlich den gewünschten Dampfdrücken über die Sättigung mit den zuvor genannten Gasen mit ihren Dampfdrücken variiert werden. Günstig dabei ist, die Sättigung mit Gasen bei Raumtemperatur oder 37°C vorzunehmen.

Das PFCL oder ein mit einem oder mehreren der oben genannten Gase gesättigtes PFCL wird, wie zuvor beschrieben, bei Raumtemperatur in das Auge gespritzt. Das Injektionsvolumen beträgt beim Menschen etwa 8 µl, füllt also bei einem Gesamtvolumen des Glaskörpers von 4000 µl nur 0,2 % dieses Raumes aus. Im Glaskörperraum wird die PFC-Flüssigkeit auf 37°C Körpertemperatur erwärmt und geht allmählich in die Gasphase über, wobei ein Augendruck von 30 mm Hg langfristig nicht überschritten werden darf. Das so injizierte PFCL verbleibt als Gasblase über Wochen im Glaskörperraum und wird allmählich über die vascularisierten Augenhüllen resorbiert. Bei den genannten PFCLs bzw. mit Gasen gesättigten PFCLs hat sich die PFCL-Gastamponade nach fünf Wochen spontan resorbiert.

## Patentansprüche

1. Verwendung eines Perfluorcarbons, das unter Normalbedingungen flüssig ist und nach Injektion in ein Auge in Folge seines Eigendampfdrucks in den Dampfzustand übergeht, zur Herstellung eines für die Injektion in den Glaskörperraum des nichtvitrektomierten Auges bestimmten Verdrängungsmittels mit einem Injektionsvolumen, das zur vollständigen Beseitigung des Wasseranteils aus dem Glaskörperraum am nichtvitrektomierten Auge durch allmähliche Bildung einer den Glaskörperraum vollständig ausfüllenden Gasblase aus dem injizierten Perfluorcarbon bei Körpertemperatur als Tamponade des Glaskörperraumes führt.

2. Verwendung nach Anspruch 1, **dadurch gekennzeichnet, dass** das Injektionsvolumen etwa 8 µl beträgt.

3. Verwendung nach einem der Ansprüche 1 oder 2, **dadurch gekennzeichnet, dass** als Perfluorcarbon Perfluor-n-pentan und/oder Perfluor-n-hexan und/oder Perfluor-n-heptan und/oder Perfluor-methylcyclopentan verwendet wird bzw. werden.

## Claims

1. Use of a perfluorocarbon, which is liquid under normal conditions and changes to the vapour state after injection in an eye due to its intrinsic vapour pressure, for the manufacture of a displacer for the injection into the vitreous body cavity of the non-vitrectomized eye having an injection volume, leading to the complete elimination of the water portion from the vitreous body cavity of the non-vitrectomized eye by gradual formation of a gas bubble from the injected perfluorocarbon completely filling the vitreous body cavity as a tamponade of the vitreous body cavity at body temperature.

2. Use according to claim 1,
**characterized in that** the injection volume is about 8 µl.

3. Use according to any of the claims 1 or 2,
**characterized in that** perfluoro-n-pentane and/or perfluoro-n-hexane and/or perfluoro-n-heptane and/or perfluoro-methylcyclopentane is/are used as the perfluorocarbon.

## Revendications

1. Utilisation d'un hydrocarbure perfluoré liquide dans des conditions normales, qui passe à l'état de vapeur lors de l'injection dans un oeil du fait de sa pression de vapeur propre, pour la fabrication d'un produit de substitution destiné à l'injection dans la cavité du corps vitreux d'un oeil n'ayant pas subi de vitrectomie, avec un volume d'injection provoquant l'élimination complète de l'eau de la cavité du corps vitreux de l'oeil n'ayant pas subi de vitrectomie grâce à la formation progressive d'une bulle de gaz, constituée de l'hydrocarbure perfluoré, remplissant complètement la cavité du corps vitreux à la température corporelle, comme tamponnement de la cavité du corps vitreux.

2. Utilisation selon la revendication 1, **caractérisée en ce que** le volume d'injection est d'environ 8 µl.

3. Utilisation selon l'une des revendications 1 ou 2, **caractérisée en ce que** l'hydrocarbure perfluoré utilisé peut être du perfluoro- n- pentane et/ou du perfluoro- n- hexane et/ou du perfluoro- n- heptane et du perfluoro- n- méthylcyclopentane.
